(19) Europäisches Patentamt
European Patent Office
Office européen des brevets

(11) Publication number: 0 034 462 B1

(12) **EUROPEAN PATENT SPECIFICATION**

(45) Date of publication of patent specification: 07.12.83

(51) Int. Cl.³: C 07 C 143/86, A 61 K 31/095

(21) Application number: 81300548.5

(22) Date of filing: 11.02.81

# (54) Imidodisulfamide derivatives, their preparation and use in pharmaceutical compositions.

(30) Priority: 13.02.80 US 121066

(43) Date of publication of application: 26.08.81 Bulletin 81/34

(45) Publication of the grant of the patent: 07.12.83 Bulletin 83/49

(84) Designated Contracting States: AT BE CH DE FR GB IT LI LU NL SE

(56) References cited:

CHEMISCHE BERICHTE, Vol. 101, No. 5, 1968 Weinheim R. APPEL et al. "Symmetrisch di- und tetra-N-substituierte Imidobis-sulfonamide" pages 1743 to 1745

(73) Proprietor: SMITHKLINE BECKMAN CORPORATION
1 Franklin Plaza
Philadelphia Pennsylvania 19101 (US)

(72) Inventor: Ali, Fadia Elfehail
5 Green Acre Drive, N.
Cherry Hill New Jersey 08003 (US)
Inventor: Krell, Robert Donald
38 Thunderhead Drive
Medford New Jersey 08055 (US)
Inventor: Snader, Kenneth Means
39 Home Road
Hatboro Pennsylvania 19040 (US)

(74) Representative: Johnston, Walter Paul
P.O. Box 39
Welwyn Garden City Hertfordshire AL7 1EY (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 034 462 B1

Imidodisulfamide derivatives their preparation and use in pharmaceutical compositions

This invention relates to certain imidodisulfamide derivatives to process for their preparation and to pharmaceutical compositions containing them.

It has been suggested that the slow reacting substance of anaphylaxis (SRS—A) released from cells e.g. mast cells which are activated by antibody-antigen combination mediates allergic reactions in man.

We have now discovered certain imidodisulfamide derivatives which antagonize the effects of SRS—A and are therefore useful in the treatment of allergic diseases for example bronchial asthma, rhinitis, hayfever and allergic eczema whose symptoms are controlled by this mediator.

Although certain imidosulfamide derivatives have been reported in Chemische Berichte Vol. 101, No. 5, 1968, they were not reported as having SRS—A antagonist activity.

Accordingly the present invention provides compounds of formula (I):—

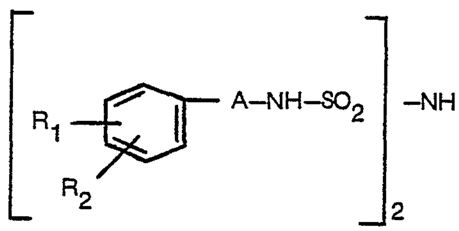

(I)

and pharmaceutically acceptable salts thereof where A represents a covalent bond (—), an alkylene chain $—(CH_2)_n—$ in which n is a positive integer 2, 3, 4 or 6, a branched alkylene chain of from 2 to 4 carbon atoms, or phenylmethylene

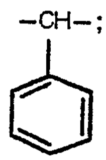

$R_1$ represents hydrogen, 3- or 4-bromo, 3- or 4-chloro, or 3- or 4-trifluoromethyl; and

$R_2$ represents hydrogen, with the proviso that when n is 2, $R_1$ is 4-chloro, 4-bromo, 4-methoxy or 3-trifluoromethyl and $R_2$ is hydrogen or together with $R_1$ in adjacent positions forms either a 3,4-dichloro substitution or a fused benzo ring, and further with the proviso that when A is a single valence bond $R_1$ is not hydrogen or 4-chloro.

Particular compounds of this invention represented by formula I above are when A is an alkylene chain, $R_1$ is hydrogen, 3-bromo, 3-chloro or 4-chloro, and $R_2$ is hydrogen.

Compounds of formula (I) are acidic and form pharmaceutically acceptable salts. Examples of such salts are alkali metal salts particularly the sodium or potassium salt.

The compounds of this invention can be prepared by reacting an amine of formula (II):—

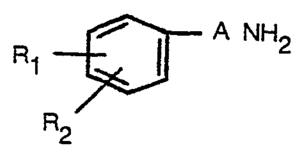

(II)

wherein $R_1$, $R_2$ and A are as defined with reference to formula (I) with bis(chlorosulfonyl)imide in the presence of a non-nucleophilic organic base.

Examples of non-nucleophic organic bases include tertiary $C_{1-4}$ alkylamines particularly triethylamine, tertiary alkylaryl amines, in particular N,N-dimethyl aniline, aromatic amines in particular pyridine and bicyclic amines in particular 1,5-diazobicyclo [4.3.0] non-5-ene and 1,5-diazabicyclo [5.4.0] undec 5-ene.

Generally the reaction is carried out in an inert polar solvent the choice of which is not critical provided that it is substantially inert to the reagents and product. One suitable solvent is acetonitrile.

The reaction is usually carried out at moderate to low temperatures. For example, the reagents are usually mixed at temperatures of 0°C. or less and the reaction is allowed to warm gradually to ambient temperature.

The reaction time is dependant on *inter alia* the particular starting materials, solvent and reaction temperature. Generally the reaction will be allowed to proceed for at least 12 hours.

The reaction product can be isolated by standard methods for example addition of dilute mineral acid e.g. hydrochloric acid, to the reaction mixture affords the compounds of formula (I) as the "free acid".

Compounds of formula (I) can be converted into pharmaceutically acceptable salts by analogy with known methods. For example alkali metal salts can be prepared by reacting the "free acid" with a solution of an alkali metal alkoxide in the corresponding alkanol (e.g. sodium methoxide in methanol).

The amines of formula (II) can be prepared by analogy with known methods. Bis(chlorosulfonyl)imide can be prepared from chlorosulfonic acid and chlorosulfonylisocyanate.

The SRS—A antagonist activity of the compounds of this invention is measured by the ability of the active medicament to inhibit SRS—A induced contraction of guinea pig ileum. In this test system, sections of ileum are resected from guinea pigs and placed in 5 ml. tissue baths containing a modified Tyrode's solution. One end of the tissue is fixed to a glass tissue holder, the other is connected to a force-displacement transducer and the tissue is placed under a tension of 500 mg. Isometric tissue contractions are recorded on a six channel polygraph. Baths are constantly aerated with 95% $O_2$—5% $CO_2$. After a 20 minute stabilization period a concentration of the appropriate agonist which provides a contraction height of 60—80% of the maximum obtainable to that agonist (as determined from full sequential concentration — response curves in separate experiments) is added to the tissue bath and the response recorded. The procedure is repeated until reproducible responses are obtained. For most agonists, two applications in rapid succession, followed 15 minutes later by a third, is sufficient to establish reproducibility. Experimental tissues are incubated with the selected concentration of the test compounds for 15 minutes. Experimental and control tissues are subjected to 5 bath changes during the incubation interval. Changes in bath fluid during the incubation period are helpful in insuring the reprodubility of tissue responses to the agonist. The same concentration of the agonist is reapplied in the presence of the test compound and the response registered and compared with controls. Percent inhibition produced by the test compound is calculated by subtracting the mean percentage change in control tissue from the mean percentage change in tissues exposed to the test compound. Additional compounds are then evaluated as long as the tissue remains reproducibly responsive to the agonist. Six tissues obtained from 6 animals are used simultaneously — 3 controls and 3 experimental.

The compounds of this invention tested at concentrations of from $5 \times 10^{-5}$ M to $1 \times 10^{-5}$ M produce marked antagonism of partially purified slow reacting substance of anaphylaxis obtained from guinea pig lung. The agonist is employed at a concentration of 40 $\mu$g/ml. For example, 1,5-di(4-bromophenyl)-imidodisulfamide produced 47% antagonism of SRS—A at $5 \times 10^{-5}$ M; 1,5,-di-(4-chlorophenethyl)imidodisulfamide produced 64% antagonism of SRS—A at $5 \times 10^{-5}$ M; and 1,5-di-(4-phenylbutyl)- imidodisulfamide produced 40% antagonism of SRS—A at $1 \times 10^{-5}$ M.

The specificity of the antagonist activity of the compounds of this invention is demonstrated by relatively low levels of antagonism toward agonists such as potassium chloride, serotonin, histamine and the prostaglandins $F_{2\alpha}$ and $E_2$.

In order to use compounds of formula (I) or salts thereof for medical purposes, they are formulated in accordance with standard pharmaceutical practice as pharmaceutical compositions.

Accordingly the invention further provides pharmaceutical compositions suitable for parenteral or topical administration comprising a compound of formula (I) above of a pharmaceutically acceptable salt thereof and a pharmaceutically acceptable carrier.

Compounds of the invention can be administered topically or systemically.

Topical formulations for administration to the skin include lotions and creams. Topical formulations for administration to the respiratory tract include solutions and suspensions for application *via* a nubulizer or as an aerosol, snuffs and microfine insufflatable powders for administration from an inhalation device.

Aerosol formulations typically comprise a suspension or solution of a compound of the invention in a conventional liquid propellant for example dichlorodifluoromethane or chlorotrifluoroethane. Such formulation are conventionally dispensed in a pressurized container, which is typically fitted with a metered dispensing valve.

The active ingredient in an insufflatable powder has a small particle size i.e. less than 50 microns and preferably less than 10 microns. The active material is co-presented with a solid carrier such as lactose which has a particle size of less than 50 microns. Such formulations are typically filled into puncturable gelatine capsules for use in an inhalation device.

Systemic administration may be achieved by parenteral administration. Typicaly parenteral compositions comprise a solution or suspension of the active material in a sterile aqueous carrier or parenterally acceptable oil (for example peanut oil or olive oil).

Preferably the composition is in unit dose form such as metered aerosol so that the patient may administer to himself a single dose.

Where appropriate, small amounts of anti-asthmatics and bronchodilators for example sympathomimetic amines such as isoprenaline, isoetharine, salbutamol, phenylephrine and ephedrine; xanthine derivatives such as theophylline and aminophylline; and corticosteroids such as

prednisolone and adrenal stimulants such as ACTH may be included.

In use, the compositions of the invention can be administered either prophylactically to prevent the on set of an allergic/asthamtic reaction or when the reaction is taking place. The precise dose of the compound of the invention administered depends *inter alia* on the age and weight of the patient and the severity of the condition. This can be determined by routine experimentation. Typically 0.5 to 2000 mg of the compound of formula (I) (or the salt calculated as the free acid) will be administered per day to an adult in from 1 to 4 doses each containing from 0.5 to 500 mg.

The following Examples illustrate the invention.

## Example 1

A mixture of 166 g. (1.42 mole) of chlorosulfonic acid and 202 g. (1.42 mole) of chlorosulfonylisocyanate was heated under reflux (110°C.) in an oil bath until the evolution of carbon dioxide ceased. The crude product was distilled *in vacuo* to yield bis(chlorosulfonyl)imide, b.p., (1.5 mm.) 100°C.

To 8.56 g. (0.04 mole) of bis(chlorosulfonyl)imide in 20 ml. of dry acetonitrile at —40°C. was added dropwise 12.1 g. (0.12 mole) of triethylamine (sodium hydroxidedried). The mixture was warmed to 0°C. and 15.48 g. (0.09 mole) of 3-bromoaniline in 20 ml. of dry acetonitrile was added slowly. The reaction mixture was stirred at room temperature for 12 hours. The hydrochloride salt of triethylamine was filtered and the filtrate was concentrated to dryness under reduced pressure. The residual oil was treated with ethanol/dilute hydrochloric acid to give a solid which was recrystallized from ethanol to furnish 1,5-di-(3-bromophenyl)-imidodisulfamide, m.p. 178—179°C.

| Analysis | C | H | N | S | Halogen |
|---|---|---|---|---|---|
| Calculated: | 29.71 | 2.29 | 8.66 | 13.22 | 32.94 |
| Found: | 29.87 | 2.36 | 8.75 | 13.30 | 32.66 |

Similarly, reacting 2.75 g. (0.0129 mole) of bis(chlorosulfonyl)imide, 5.38 ml. (0.0386 mole) of triethylamine and 5.0 g. (0.029 mole) of 4-bromoaniline as described above gave 1,5-di-(4-bromophenyl)-imidodisulfamide, m.p. 215—222°C (sodium salt).

| Analysis | C | H | N |
|---|---|---|---|
| Calculated: | 27.42 | 2.28 | 8.00 |
| Found: | 27.52 | 2.19 | 8.43 |

## Example 2

To 17.12 g. (0.08 mole) of bis(chlorosulfonyl)imide in 40 ml. of dry acetonitrile at —40°C. was added dropwise 16.12 g. (0.16 mole) of dry triethylamine. The mixture was warmed to 0°C., 20.4 g. (0.16 mole) of 3-chloroaniline in 40 ml. of dry acetonitrile was added slowly and the resultant mixture was stirred at room temperature for 12 hours. The reaction mixture was filtered and the filtrate was concentrated. The residue was dissolved in methanol, acidified with dilute hydrochloric acid and the resulting solid was recrystallized from aqueous methanol to give 1,5-di-(3-chlorophenyl)-imidodisulfamide, m.p. 164.5—166°C.

| Analysis | C | H | N | S | Halogen |
|---|---|---|---|---|---|
| Calculated: | 35.96 | 2.89 | 10.49 | 16.00 | 17.69 |
| Found: | 36.08 | 3.19 | 10.68 | 15.78 | 17.78 |

Following the procedures of Examples 1 and 2, the appropriately substituted primary amines were reacted with bis(chlorosulfonyl)imide in the presence of triethylamine to give the imidodisulfamide product as indicated in the following table:

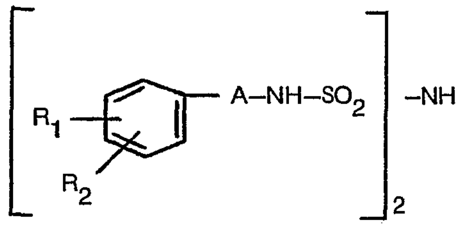

(I)

| Example | $R_1$ | $R_2$ | A | M.P. °C. | | C | H | N | S | Halo |
|---|---|---|---|---|---|---|---|---|---|---|
| | | | | | | ANALYSIS | | | | |
| 3 | 3—$CF_3$ | H | — | 179—80 | Calculated | 36.29 | 2.39 | 9.07 | 13.84 | 24.60 |
| | | | | | Found | 36.57 | 2.51 | 9.16 | 14.06 | 24.32 |
| 4 | 4—$CF_2$ | H | — | 148.5—9 | Calculated | 36.29 | 2.39 | 9.07 | 13.84 | |
| | | | | | Found | 35.96 | 2.55 | 9.07 | 12.98 | |
| 5 | 4—Cl | H | $(CH_2)_2$ | 148—9 | Calculated | 42.48 | 4.23 | 9.29 | 14.18 | 15.67 |
| | | | | | Found | 42.22 | 3.94 | 9.29 | 13.96 | 15.30 |
| 6 | 4—Br | H | $(CH_2)_2$ | 152—4 | Calculated | 35.50 | 3.54 | 7.76 | | |
| | | | | | Found | 35.43 | 3.39 | 7.97 | | |
| 7 | 3—$CF_3$ | H | $(CH_2)_2$ | 139—40 | Calculated | 41.62 | 3.61 | 8.09 | | |
| | | | | | Found | 41.49 | 3.72 | 8.19 | | |
| 8 | 4—$OCH_3$ | H | $(CH_2)_2$ | 152—3 | Calculated | 48.74 | 5.68 | 9.47 | 14.46 | |
| | | | | | Found | 48.74 | 5.86 | 9.41 | 14.17 | |
| 9 | 4—Cl | 3—Cl | $(CH_2)_2$ | 110—11 | Calculated | 36.88 | 3.29 | 8.06 | | |
| | | | | | Found | 37.01 | 3.56 | 7.57 | | |
| 10 | 2,3-benzo | | $(CH_2)_2$ | 168—70 | Calculated | 58.52 | 5.32 | 8.53 | 13.02 | |
| | | | | | Found | 58.38 | 5.33 | 8.22 | 12.97 | |

ANALYSIS

| Example | $R_1$ | $R_2$ | A | M.P. °C | | C | H | N | S | Halo |
|---|---|---|---|---|---|---|---|---|---|---|
| 11 | H | H | $CH(CH_3)CH_2$ | 80—85 | Calculated | 52.53 | 6.12 | 10.21 | 15.58 | |
| | | | | | Found | 52.59 | 6.28 | 10.19 | 15.30 | |
| 12 | H | H | $(CH_2)_2CH(CH_3)$ | 143—50 | Calculated | 54.65 | 6.65 | 9.56 | 14.59 | |
| | | | | | Found | 54.58 | 6.73 | 9.48 | 14.45 | |
| 13 | H | H | $CH(C_6H_5)$ | 198—200 | Calculated | 61.52 | 4.96 | 8.28 | 12.63 | |
| | | | | | Found | 61.26 | 5.09 | 8.26 | 12.47 | |
| 14 | 4—Cl | H | $CH(CH_3)CH_2$ | 146—8 | Calculated | 45.00 | 4.83 | 8.75 | 13.32 | 14.76 |
| | | | | | Found | 45.36 | 4.98 | 8.75 | 13.16 | 14.38 |
| 15 | 4—Cl | H | $CH(CH_3)$ | 151—3 | Calculated | 42.48 | 4.23 | 9.29 | 14.18 | 15.67 |
| | | | | | Found | 42.38 | 4.32 | 9.31 | 14.20 | 15.88 |
| 16 | H | H | $(CH_2)_3$ | 174—5 | Calculated | 52.53 | 6.12 | 10.21 | 15.58 | |
| | | | | | Found | 52.61 | 6.13 | 10.24 | 15.38 | |
| 17 | H | H | $(CH_2)_4$ | 133—4 | Calculated | 54.65 | 6.65 | 9.56 | 14.59 | |
| | | | | | Found | 54.59 | 6.54 | 9.60 | 14.34 | |
| 18 | H | H | $(CH_2)_6$ | 134—5 | Calculated | 58.15 | 7.52 | 8.48 | 12.94 | |
| | | | | | Found | 58.10 | 7.45 | 8.54 | 13.12 | |
| 19 | 4—Cl | H | $(CH_2)_4$ | 124.5—125.5 | Calculated | 47.24 | 5.35 | 8.26 | 12.61 | 13.94 |
| | | | | | Found | 47.63 | 5.50 | 8.40 | 12.90 | 13.54 |

Exemplary of the preparation of the required primary amine starting materials is the following preparation of 4-(p-chlorophenyl)-butylamine:

A mixture of 4-(p-chlorophenyl)-butyric acid (6.7 g., 0.0337 mole) in 50 ml. of dry chloroform and 10 ml. (0.134 mole) of thionyl chloride was refluxed for 8 hours. Excess thionyl chloride was removed *in vacuo* and the residual oil was treated with toluene to remove traces of chloride. The residue was taken up in 10 ml. of toluene and added to 30 ml. of concentrated ammonium hydroxide in an ice-water bath to give 4-(p-chlorophenyl)-butyramide, m.p. 110—113°C.

To a solution of 12.6 g (0.33 mole) of lithium aluminum hydride in 340 ml. of dry ether was added dropwise at room temperature 16.4 g. (0.083 mole) of 4-(p-chlorophenyl)-butyramide. The reaction mixture was stirred at room temperatures for 4 hours, cooled and cautiously quenched with 13 ml. of water, 10 ml. of 20% sodium hydroxide and 45 ml. of water. The organic layer was separated and the emulsion was extracted with ether. The combined organic and ether extract was dried over sodium hydroxide and concentrated to obtain 4-(p-chlorophenyl)butylamine; m.p. of hydrochloride salt 162—164°C.

As a specific embodiment of a composition of this invention, an active ingredient such as 1,5-di-(4-phenylbutyl)-imidodisulfamide is dissolved in sterile water at a concentration of 0.5% and aerosolized from a nebulizer operating at an air flow adjusted to deliver the desired aerosolized weight of drug.

**Claims for the contracting states: BE CH/LI DE FR GB IT LU NL SE**

1. A compound of formula (I):

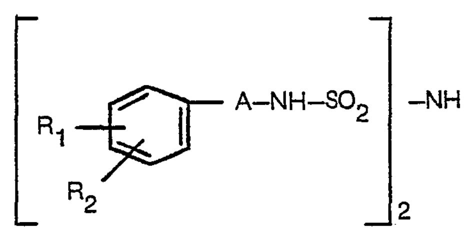

(I)

and pharmaceutically acceptable salts thereof where A is covalent bond, an alkylene chain $—(CH_2)_n—$ in which n is a positive integer 2, 3, 4 or 6, a branched alkylene chain of from 2 to 4 carbon atoms, or phenylmethylene; $R_1$ is hydrogen, 3- or 4-bromo, 3- or 4-chloro, or 3- or 4-trifluoromethyl; and

$R_2$ is hydrogen, with the proviso that when n is 2, $R_1$ is 4-chloro, 4-bromo, 4-methoxy or 3-trifluoromethyl and $R_2$ is hydrogen or together with $R_1$ in adjacent positions forms either a 3, 4-dichloro substitution or a fused benzo ring, and further with the proviso that when A is a covalent bond $R_1$ is not hydrogen or 4-chloro.

2. A compound as claimed in claim 1 in which A is an alkylene chain, $R_1$ is hydrogen, 3-bromo- 3-chloro or 4-chloro, and $R_2$ is hydrogen.

3. A compound as claimed in claim 1 in which A is a covalent bond.

4. 1,5-di-(4-chlorophenethyl)-imidodisulfamide, 1,5-di(4-phenylbutyl)-imidodisulfamide and 1,5-di-(4-bromophenyl)-imidodisulfamide.

5. A process for preparing a compound as claimed in claim 1 which comprises reacting an amine of formula (II):—

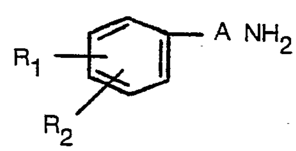

(II)

where $R_1$, $R_2$ and A are as defined with reference to formula (I) with bis(chlorosulfonyl)imide in the presence of a non-neucleophilic organic base, and optionally converting the compound of formula (I) so obtained into a salt.

6. A pharmaceutical composition suitable for parenteral or topical administration comprising a compound as claimed in any one of claims 1 to 4 and a pharmaceutically acceptable carrier.

7. A composition as claimed in claim 6 in a form suitable for topical administration to the respiratory tract.

8. A composition as claimed in claim 7 in the form of an aerosol formulation of insufflatable powder.

9. A composition as claimed in claim 6 suitable for administration by injection.

**Claims for the contracting state AT**

1. A process for preparing a compound of formula (I):—

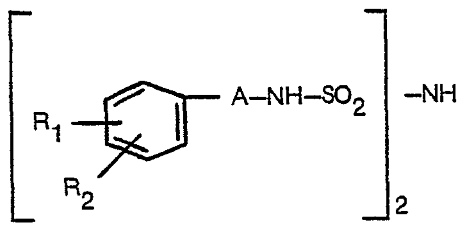

(I)

and pharmaceutically acceptable salts thereof where A is a covalent bond, an alkylene chain $—(CH_2)_n—$ in which n is a positive integer 2, 3, 4, or 6, a branched alkylene chain of from 2 to 4 carbon atoms, or phenylmethylene; $R_1$ is hydrogen, 3- or 4-bromo, 3- or 4-chloro, or 3- or 4-trifluoromethyl; and

$R_2$ is hydrogen, with the proviso that when n is 2, $R_1$ is 4-chloro, 4-bromo, 4-methoxy or 3-trifluoromethyl and $R_2$ is hydrogen or together with $R_1$ in adjacent positions forms either a 3,4-dichloro substitution or a fused benzo ring, and further with the proviso that when A is a covalent bond $R_1$ is not hydrogen or 4-chloro; which comprises reacting an amine of formula (II):—

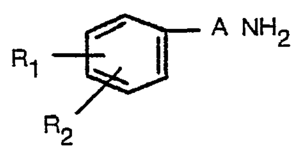

(II)

where $R_1$, $R_2$ and A are as defined with reference to formula (I) with bis(chlorosulfonyl)imide in the presence of a non-neucleophilic organic base, and optionally converting the compound of formula (I) so obtained into a salt.

2. A process as claimed in claim 1 in which A is an alkylene chain, $R_1$ is hydrogen, 3-bromo, 3-chloro or 4-chloro, and $R_2$ is hydrogen.

3. A process as claimed in claim 1 in which A is a covalent bond.

**Revendications pour les etats contractants: BE, CH, LI, DE, FR, GB, IT, LU, NL, SE**

1. Composé de formule (I):

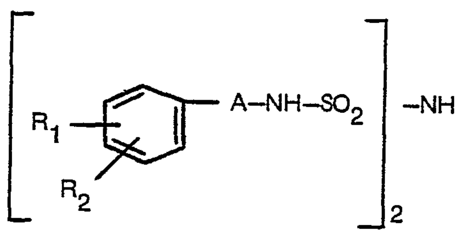

(I)

et les sels pharmaceutiquement acceptables de ce composé dans lequel A représente une liaison covalente=, une chaîne alcoylène$—(CH_2)_n—$ dans laquelle n est un nombre entier positif égal à 2, 3, 4 ou 6, une chaîne alcoylène ramifiée de 2 à 4 atomes de carbone ou un groupe phénylméthylène; $R_1$ représente de l'hydrogène, un groupe 3- ou 4- bromo, 3- ou 4-chloro ou 3- ou 4-trifluorométhyle; et

$R_2$ représente de l'hydrogène, à condition que lorsque n vaut 2, $R_1$ représente un groupe 4-chloro, 4-bromo, 4-méthoxy ou 3-trifluorométhyle et $R_2$ représente de l'hydrogène ou, avec $R_1$ en positions adjacentes, $R_2$ forme soit une substitution 3,4-dichloro soit un noyau benzocondensé et, en outre, à condition que lorsque A représente une liaison covalente, $R_1$ ne représente pas de l'hydrogène ou un groupe 4-chloro.

2. Composé tel que revendiqué dans la revendication 1, dans lequel A représente une chaîne

alcoylène, $R_1$ représente de l'hydrogène, un groupe 3-bromo, 3-chloro ou 4-chloro et $R_2$ représente de l'hydrogène.

3. Composé tel que revendiqué dans la revendication 1, dans lequel A représente une liaison covalente.

4. Le 1,5-di-(4-chlorophénéthyl)-imidodisulfamide, le 1,5-di(4-phénylbutyl)-imidodisulfamide et le 1,5-di-(4-bromophényl)-imidodisulfamide.

5. Procédé de préparation d'un composé tel que revendiqué dans la revendication 1 qui consiste à faire réagir une amine de formule (II):

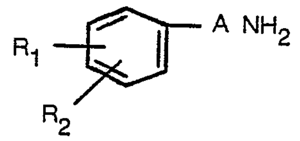

(II)

dans laquelle $R_1$, $R_2$ et A sont tels que définis à la formule I avec le bis(chlorosulfonyl)imide en présence d'une base organique non-nucléophile et à transformer éventuellement le composé de formule (I) ainsi obtenu en un sel.

6. Composition pharmaceutique applicable à l'administration parentérale ou locale qui comprend un composé tel que revendiqué dans l'une quelconque des revendications 1 à 4 et un véhicule pharmaceutiquement acceptable.

7. Composition telle que revendiquée dans la revendication 6 sous une forme applicable à l'administration locale destinée aux voies respiratoires.

8. Composition telle que revendiquée dans la revendication 7 sous la forme d'une présentation d'une poudre insufflable pour aérosol.

9. Composition telle que revendiquée dans la revendication 6 applicable à l'administration par injection.

**Revendications pour l'etat contractant: AT**

1. Procédé par préparation d'un composé de formule (I):

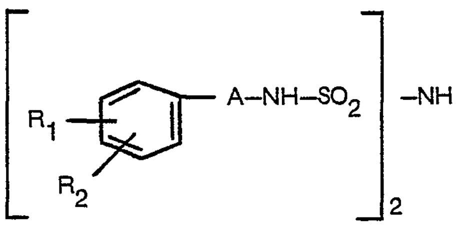

(I)

et des sels pharmaceutiquement acceptables de ce composé dans lequel A représente une liaison covalente=, une chaîne alcoylène—$(CH_2)_n$— dans laquelle n est un nombre entier positif égal à 2, 3, 4 ou 6, une chaîne alcoylène ramifiée de 2 à 4 atomes de carbone ou un groupe phénylméthylène; $R_1$ représente de l'hydrogène, un groupe 3- ou 4- bromo, 3- ou 4-chloro ou 3- ou 4-trifluorométhyle; et

$R_2$ représente de l'hydrogène, à condition que lorsque n vaut 2, $R_1$ représente un groupe 4-chloro, 4-bromo, 4-méthoxy ou 3-trifluorométhyle et $R_2$ représente de l'hydrogène ou, avec $R_1$ en positions adjacentes, $R_2$ forme soit une substitution 3,4-dichloro soit un noyau benzocondensé et, en outre, à condition que lorsque A représente un liaison covalente, $R_1$ ne représente pas de l'hydrogène ou un groupe 4-chloro; qui consiste à faire réagir une amine de formule (II):

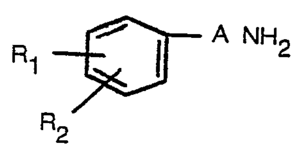

(II)

dans laquelle $R_1$, $R_2$ et A sont tels que définis à la formule I avec le bis(chlorosulfonyl)imide en présence d'une base organique non-nucléophile et à transformer éventuellement le composé de formule (I) ainsi obtenu en un sel.

2. Procédé tel que revendiqué dans la revendication 1, dans lequel A représente une chaîne alcoylène, $R_1$ représente de l'hydrogène, un groupe 3-bromo, 3-chloro ou 4-chloro et $R_2$ représente de l'hydrogène.

3. Procédé tel que revendiqué dans la revendication 1, dans lequel A représente une liaison covalente.

**Patentansprüche für die Vertragsstaaten BE CH/LI DE FR GB IT LU NL SE**

1. Eine Verbindung der Formel I:

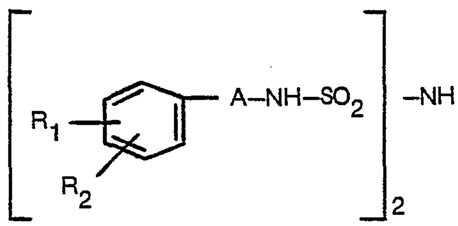

(I)

und ihre pharmazeutisch verträglichen Salze, in der A eine kovalente Bindung, eine Alkylenkette $-(CH_2)_n-$, in der n eine positive ganze Zahl 2, 3, 4 oder 6 ist, eine verzweigte Alkylenkette mit 2 bis 4 Kohlenstoffatomen oder Phenylmethylen ist; $R_1$ Wasserstoff, 3- oder 4-Brom, 3- oder 4-Chlor, oder 3- oder 4-Trifluormethyl ist; und

$R_2$ Wasserstoff ist, mit der Maßgabe, daß $R_1$ 4-Chlor, 4-Brom-4-Methoxy oder 3-Trifluormethyl ist und $R_2$ Wasserstoff ist oder zusammen mit $R_1$ in benachbarter Stellung entweder eine 3,4-Dichlorsubstitution darstellt oder einen kondensierten Benzolring bildet, wenn n 2 ist, und mit der weiteren Maßgabe, daß $R_1$ kein Wasserstoff oder 4-Chlor ist, wenn A eine kovalente Bindung ist.

2. Eine Verbindung nach Anspruch 1, in der A eine Alkylenkette ist, $R_1$ Wasserstoff, 3-Brom, 3-Chlor oder 4-Chlor ist und $R_2$ Wasserstoff ist.

3. Eine Verbindung nach Anspruch 1, in der A eine kovalente Bindung ist.

4. 1,5-Di-(4-chlorphenäthyl)-imidodisulfamid, 1,5-Di-(4-phenylbutyl)-imidodisulfamid und 1,5-Di-(4-Bromophenyl)-imidodisulfamid.

5. Ein Verfahren zur Herstellung einer Verbindung nach Anspruch 1, gekennzeichnet durch Umsetzung eines Amins der Formel II

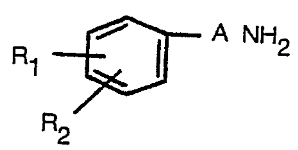

(II)

in der $R_1$, $R_2$ und A wie bezüglich der Formel I definiert sind, mit Bis(chlorsulfonyl)imid in Gegenwart einer nichtnucleophilen organischen Base und gegebenenfalls durch Umwandlung der so erhaltenen Verbindung der Formel I in ein Salz.

6. Ein Arzneimittel, geeignet für parenterale oder lokale Anwendung, enthaltend eine Verbindung nach einem der Ansprüche 1 bis 4 und einen pharmazeutisch verträglichen Träger.

7. Ein Mittel nach Anspruch 6 in einer Form, die geeignet ist zur lokalen Behandlung der Atmungswege.

8. Ein Mittel nach Anspruch 7 in der Form eines Aerosolpräparats aus einblasbarem Pulver.

9. Ein Mittel nach Anspruch 6, geeignet zur Anwendung durch Injektion.

**Ansprüche für den Vertragsstaat AT**

1. Ein Verfahren zur Herstellung einer Verbindung der Formel I:

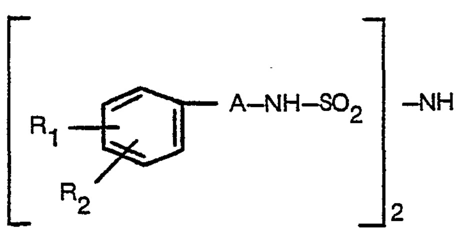

(I)

und ihre pharmazeutisch verträglichen Salze, in der A eine kovalente Bindung, eine Alkylenkette $—(CH_2)_n—$, in der n eine positive ganze Zahl 2, 3, 4 oder 6 ist, eine verzweigte Alkylenkette mit 2 bis 4 Kohlenstoffatomen oder Phenylmethylen ist; $R_1$ Wasserstoff, 3- oder 4-Brom, 3- oder 4-Chlor oder 3- oder 4-Trifluormethyl ist; und

$R_2$ Wasserstoff ist, mid der Maßgabe, daß $R_1$ 4-Chlor, 4-Brom, 4-Methoxy oder 3-Trifluormethyl ist und $R_2$ Wasserstoff ist oder zusammen mit $R_1$ in benachbarter Stellung entweder eine 3,4-Dichlorsubstitution darstellt, oder einen kondensierten Benzolring bildet, wenn n 2 ist, und mit der weiteren Maßgabe, daß $R_1$ kein Wasserstoff oder 4-Chlor ist, wenn A eine kovalente Bindung ist, gekennzeichnet durch Umsetzung eines Amins der Formel II;

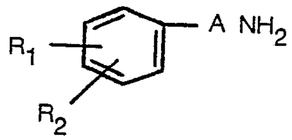

(II)

in der $R_1$, $R_2$ und A wie bezüglich der Formel I definiert sind, mit Bis(chlorsulfonyl)imid in Gegenwart einer nicht-nukleophilen organischen Base und gegebe nenfalls durch Umwandlung der so erhaltenen Verbindung der Formel I in ein Salz.

2. Ein Verfahren nach Anspruch 1, wobei A eine Alkylenkette ist, $R_1$ Wasserstoff, 3-Brom, 3-Chlor oder 4-Chlor ist und $R_2$ Wasserstoff ist.

3. Ein Verfahren nach Anspruch 1, wobei A eine kovalente Bindung ist.